Europäisches Patentamt

European Patent Office

Office européen des brevets

(19)

(11) Veröffentlichungsnummer: **0 198 407 B1**

(12) **EUROPÄISCHE PATENTSCHRIFT**

(45) Veröffentlichungstag der Patentschrift: **24.07.91**

(51) Int. Cl.5: **A61M 1/28**

(21) Anmeldenummer: **86104834.6**

(22) Anmeldetag: **09.04.86**

(54) **Konnektor für Peritonealdialyse.**

(30) Priorität: **12.04.85 DE 3513205**

(43) Veröffentlichungstag der Anmeldung:
**22.10.86 Patentblatt 86/43**

(45) Bekanntmachung des Hinweises auf die
Patenterteilung:
**24.07.91 Patentblatt 91/30**

(84) Benannte Vertragsstaaten:
**AT BE CH DE FR GB IT LI LU NL SE**

(56) Entgegenhaltungen:
**EP-A- 0 029 526       EP-A- 0 080 379**
**EP-A- 0 116 986       DE-A- 3 100 622**
**US-A- 4 187 846       US-A- 4 306 976**

(73) Patentinhaber: **Fresenius AG**
**Gluckensteinweg 5**
**W-6380 Bad Homburg v.d.H.(DE)**

(72) Erfinder: **Mathieu, Bernd, Dr. Dipl.-Chem.**
**Galgenbergstrasse 11**
**W-6683 Spiesen-Elversberg(DE)**

(74) Vertreter: **Dr. Fuchs, Dr. Luderschmidt**
**Dipl.-Phys. Seids, Dr. Mehler Patentanwälte**
**Abraham-Lincoln-Strasse 7**
**W-6200 Wiesbaden(DE)**

**Beschreibung**

Die Erfindung betrifft einen Konnektor für eine CAPD-Schlauchverbindung mit einem männlichen Konnektorstück, das ein zentrales Rohrstück aufweist, das von einer Schutzhülse umfaßt und übergriffen ist, und einem weiblichen Konnektorstück, das eine Ventileinrichtung aufweist, welche im ungekuppelten Zustand eine Fluidsperre darstellt, wobei der Einführungsabschnitt in einen Aufnahmebereich der Schutzhülse mit einer Dichtung zwischen der Schutzhülse und dem weiblichen Konnektorstück flüssigkeitsdicht einführbar ist, mit einem ersten Anschluß am männlichen Konnektorstück für einen vollen Beutel mit Dialysierflüssigkeit, wobei der erste Anschluß mit dem zentralen Rohrstück in Fluidverbindung steht, mit einem zweiten Anschluß am zweiten Konnektorstück, an welchem ein Peritonealkatheter anschließbar ist, und mit einer Verbindungseinrichtung zur Sicherung einer Konnektionsstellung zwischen dem weiblichen und dem männlichen Konnektorstück.

Ein derartiger Konnektor ist beispielsweise im Deutschen Gebrauchsmuster 78 36 790 oder der EP-A- 116 986 beschrieben. Ein solcher Katheter wird bei der kontinuierlichen ambulanten Peritonealdialyse (CAPD) eingesetzt, bei welcher ein Katheter ständig im Peritonealraum eines Patienten verbleibt. Ein solcher Katheter steht durch die Bauchwand des Patienten vor und wird mit einem Teil des Konnektors verbunden und der andere Teil des Konnektors ist über eine Schlauchleitung mit einem Dialysierflüssigkeits enthaltenden Beutel verbunden. Im konnektierten Zustand kann die Dialysierflüssigkeit dem Patienten zur Entfernung der Stoffwechselprodukte zugeführt und auch wieder vom Patienten entfernt werden.

Problematisch bei dieser Dialysier-Behandlung ist das Austauschen eines Beutels mit gebrauchter Dialysierflüssigkeit durch einen Beutel mit frischer Dialysierflüssigkeit, da der Konnektor geöffnet werden muß, was unter absolut sterilen Bedingungen zu erfolgen hat. Insofern muß der Patient den Konnektor mit größter Sorgfalt öffnen, wobei der gebrauchte Beutel verworfen und durch einen frischen Beutel mit einem entsprechenden neuen sterilen Konnektorstück ersetzt wird.

Der Konnektor der eingangs erwähnten Art sieht bereits eine Schutzhülse am männlichen Konnektorteil vor, wodurch die entsprechenden Verbindungsflächen des Konnektors nicht mehr mit den Fingern berührt werden können. Desweiteren ist im konnektierten Zustand zwischen den Konnektorteilen ein mit Desinfektionsmittel zu füllender Raum vorgesehen, wodurch dennoch erzeugte Kontaminationen beseitigt werden sollen. Dieser bekannte Konnektor kann jedoch nur von besonders geschulten Patienten eingesetzt werden, die entsprechend sorgfältig und genau diese Maßnahmen durchführen. Bei älteren oder schwächlichen Patienten sind jedoch die Handhabungsprobleme mit diesem Konnektor nicht zu übersehen, so daß nach weiteren Lösungen dieses Problems gesucht wurde.

So wurde beispielsweise ein Beutelsystem für die Peritoneal-Dialyse in der EP-A- 29 526 vorgeschlagen, bei dem vom Peritonealkatheter oder vom Verbindungschlauch y-förmig ein weiterer Schlauch abzweigt, der mit einem weiteren Beutel verbunden ist. Sämtliche Schlauchstücke können durch Klemmen verschlossen werden, so daß bestimmte Fluid-Passagen geöffnet bzw. geschlossen werden können. So kann Dialysierflüssigkeit vom vollen Beutel durch den Konnektor in den Peritonealraum, vom vollen Beutel durch den Konnektor in den Leerbeutel und vom Peritonealraum in den Leerbeutel befördert werden.

Dieses bekannte System mit y-förmigen Verzweigung weist jedoch den Nachteil auf, daß einfache Stecker für einen Konnektor verwendet werden, die nicht kontaminationssicher sind; zudem befindet sich die y-förmige Verzweigung in einem der Schlauchteile, so daß hierdurch auch Flüssigkeitsreste, die u. U. kontaminiert bzw. für den Patienten infolge der Toxizität (Desinfektionsmittel) nicht unproblematisch sind, in den Peritonealraum des Patienten gelangen können. Weiterhin besteht die Gefahr, daß beim Öffnen des männlichen Konnektorstücks evtl. erzeugte Partikel ebenfalls in den Körper des Patienten gelangen können.

Festzustellen ist daher, daß zu einer gründlichen Spülung des männlichen Konnektorstücks bzw. des gesamten Konnektors einerseits eine sorgfältige Handhabung des Rollenklemmen notwendig ist, um den Spülvorgang ohne Gefahren für den Patienten ordnungsgemäß durchführen zu können, und andererseits eine nicht unerheblichen Menge Wasser für reine Spülzwecke verschwendet wird.

Weitere Konnektoren für med. Zwecke, nicht jedoch für den Einsatz bei der CAPD sind aus den EP-A- 135 140 und EP-A- 80 379 bekannt. Bei diesen Konnektoren kommt es letztlich nur auf einen sicheren Verschluß eines Konnektorteils im unkonnektierten Zustand, nicht jedoch auf die absolute Sterilität beim Konnektieren an.

Es ist daher Aufgabe der vorliegenden Erfindung, einen Konnektor für ein Zweibeutel -System nach dem Oberbegriff des Anspruches 1 zu schaffen, welcher frei von den oben erwähnten Nachteilen ist, das heißt, bei dem unter Verzicht auf die umständlich und kompliziert zu handhabenden Rollenklemmen eine rasche und sichere Spülung des Konnektors mit einem Minimum an Dialysierflüssigkeit möglich ist.

Die Lösung dieser Aufgabe erfolgt dadurch, daß am männlichen Konnektorstück im Bereich des ersten Anschlusses an der Schutzhülse ein dritter Anschluß für einen Leerbeutel für verbrauchte Dialysierflüssigkeit angeordnet ist und daß die Ventileinrichtung in Abhängigkeit von der Konnektionsstellung des weiblichen Konnektorstückes relativ zu dem männlichen Konnektorstück und im Zusammenwirken mit dem zentralen Rohrstück des männlichen Konnektorstücks Fluidpassagen zwischen dem ersten, zweiten und dritten Anschluß derart freigibt daß entweder der erste Anschluß mit dem zweiten Anschluß oder der erste Anschluß mit dem dritten Anschluß oder der zweite Anschluß mit dem dritten Anschluß verbunden wird, während gleichzeitig jeweils die Fluidverbindung zum übrigen Anschluß unterbrochen wird. Gemäß Anspruch 1 werden die Fluidpassagen zwischen vollem Beutel, Peritoneum und Leerbeutel in Abhängigkeit von dem Konnektionszustand des männlichen Konnektorstücks relativ zu dem weiblichen Konnektorstück den jeweiligen Erfordernissen entsprechend freigegeben und unterbrochen, wobei der Anschluß für den Leerbeutel am weiblichen Konnektor im Bereich des Anschlusses für den vollen Beutel angeordnet ist, so daß einerseits auf die Rollenklemmen verzichtet werden kann, da die Fluidpassagen allein durch entsprechende Manipulationen am Konnektor freigegeben oder unterbrochen werden, und andererseits durch den Verzicht eines Y-Stücks zwischen dem weiblichen Konnektor und dem Peritonealkatheter allein der Konnektor selbst und nicht noch dazugehörige Schlauchleitungen gespült werden müssen, so daß der Spülvorgang selbst in relativ kurzer Zeit mit einem geringen Aufwand an Dialysierflüssigkeit erfolgt.

Aus der DE-A- 28 53 635 und der US-A- 43 46 793 sind Konnektoren für die CAPD der eingangs erwähnten Art bekannt, die jeweils ein männliches und ein weibliches Konnektorstück aufweisen, die jeweils mit einer Schlauchleitung verbunden sind. Da diese Konnektoren im gekuppelten Zustand die Strömungsverbindung zwischen dem Peritonealraum und dem Beutel frei geben, muß die Schlauchleitung jeweils entsprechend durch eine Klammer abgesperrt werden, um ein unbeabsichtigtes Aus- oder Einlaufen der Dialysierflüssigkeit aus dem Beutel bzw. in den Beutel zu verhindern. Des weiteren ist der Konnektor selbst nicht vollständig von zurückgebliebenem Desinfektionsmittel zu reinigen, so daß die Gefahr besteht, das ein Rest des Desinfektionsmittels in den Peritonealraum des Patienten bei der Füllung mit frischer Dialysierflüssigkeit gelangen kann.

Die Unteransprüche haben vorteilhafte Weiterbildungen der Erfindung zum Inhalt.

Weitere Einzelheiten, Merkmale und Vorteile der vorliegenden Erfindung ergeben sich aus der nachfolgenden Beschreibung einer Ausführungsform anhand der Zeichnung.

Es zeigt:

Fig. 1 in Schnittdarstellung einen erfindungsgemäßen Konnektor mit einem männlichen Konnektorstück und einem weiblichen Konnektorstück, wobei diese beiden Konnektorstücke im nichtkonnektierten Zustand dargestellt sind;

Fig. 2 eine Draufsicht von unten auf das männliche Konnektorstück;

Fig. 3 in Schnittdarstellung die Stellung des männlichen Konnektorstücks zum weiblichen Konnektorstück in einem ersten Konnektionszustand;

Fig. 4 die Stellung des männlichen Konnektorstückes zu dem weiblichen Konnektorstück in einem zweiten Konnektionszustand;

Fig. 5 die Stellung des männlichen Konnektorstückes zu dem weiblichen Konnektorstück in einem dritten Konnektionszustand;

Fig. 6 die Stellung des männlichen Konnektorstückes zu dem weiblichen Konnektorstück in einem vierten Konnektionszustand; und

Fig. 7 eine Führungsnut am weiblichen Konnektorstück in abgewickelter Darstellung.

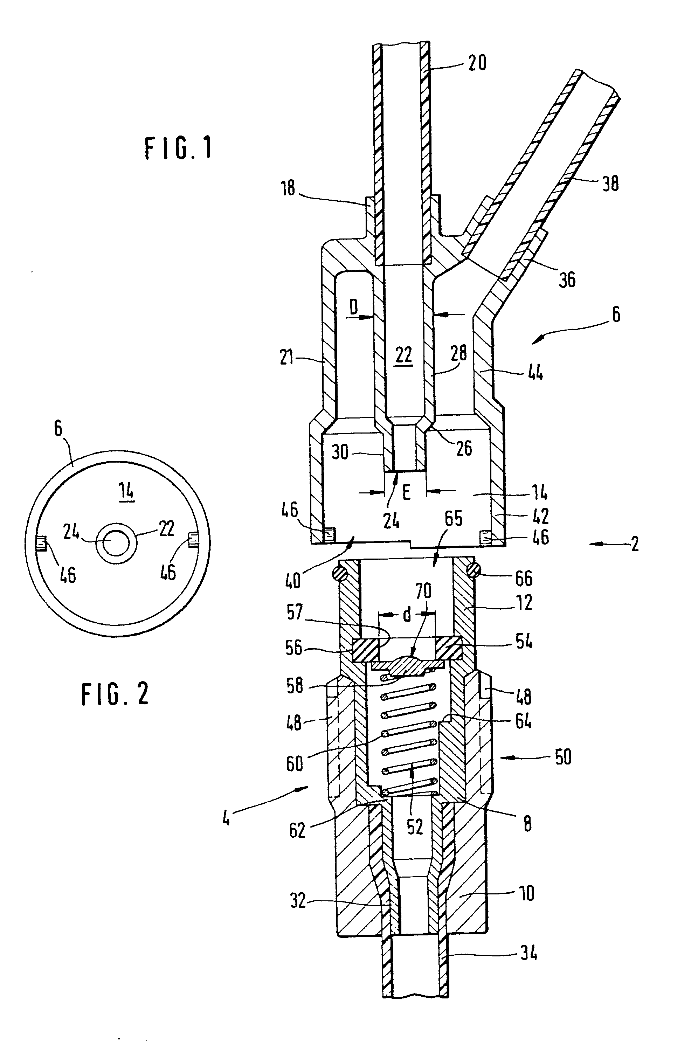

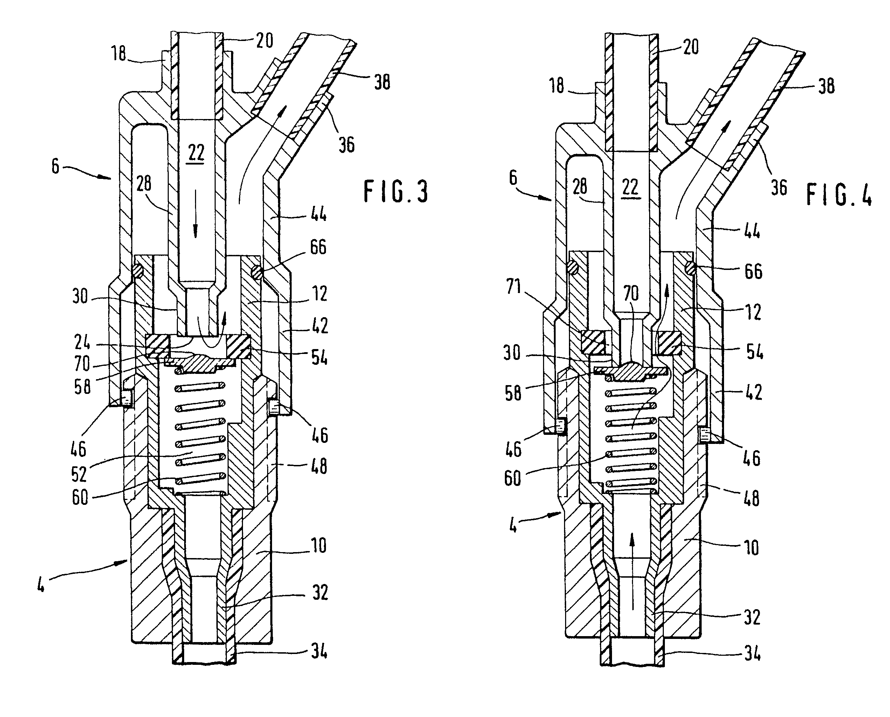

Gem. Fig. 1 weist ein Konnektor 2 ein weibliches Konnektorstück 4 und ein männliches Konnektorstück 6 auf. Das weibliche Konnektorstück 4 weist eine Konnektoreinheit 8 auf, welche in einer ersten Hülse 10 hinter einem Einführabschnitt 12 gelagert ist.

Das männliche Konnektorstück 6 weist einen Aufnahmeabschnitt 14 zur Aufnahme des weiblichen Konnektorstücks 4 auf. Weiterhin weist das männliche Konnektorstück 6 an seinem dem Aufnahmebereich 14 gegenüberliegenden Ende einen ersten Anschluß 18 für einen Schlauch 20` auf, wobei der Schlauch 20 die Verbindungsleitung zwischen einem in der Zeichnung nicht dargestellten vollen Beutel mit frischer Dialysierflüssigkeit und dem männlichen Konnektorstück 6 herstellt. Die Befestigung des Schlauches 20 an dem ersten Anschluß 18 kann beispielsweise durch Verkleben oder dergleichen erfolgen. Wie aus der Zeichnung hervorgeht, erstreckt sich der Anschluß 18 durch die Hülse 21 und geht in Richtung auf den Aufnahmebereich 14 in ein zentrales Rohrstück 22 über, das koaxial zur Hülse 21 in dem männlichen Konnektorstück 6 angeordnet ist und in seinem Frontabschnitt 30 eine Mündungsöffnung 24 aufweist.

Wie weiterhin aus der Zeichnung hervorgeht, ist der Außendurchmesser des Rohrstücks 22 über seine gesamte Länge betrachtet nicht konstant; vielmehr weist das Rohrstück 22 einen Hauptabschnitt 28 auf, der an einer Abstufung 26 in einen Frontabschnitt 30 übergeht, dessen Außendurchmesser geringer ist als derjenige des Hauptabschnitts 28. Beide Abschnitte weisen eine für die jeweiligen Konnektionsphasen bedeutsame axiale Länge auf.

Das weibliche Konnektorstück 4 weist an seinem dem Einführabschnitt 12 gegenüberliegenden Ende einen zweiten Anschluß 32 für einen zweiten Schlauch 34 auf, der zu einem in der Zeichnung nicht dargestellten Peritonealkatheter führt. Die Befestigung des zweiten Schlauches 34 an dem zweiten Anschluß 32 erfolgt beispielsweise durch eine Klemmverbindung zwischen dem zweiten Anschluß 32 und der Hülse 10, durch Aufschrumpfen des Schlauches 34 auf den zweiten Anschluß 32, durch eine Verklebung oder dergleichen.

Das männliche Konnektorstück 6 weist einen dritten von der Hülse 21 abgehenden Anschluß 36 für einen dritten Schlauch 38 auf, der eine Verbindung zwischen dem männlichen Konnektorstück 6 und einem leeren Beutel für verbrauchte Dialysierflüssigkeit darstellt. Dieser dritte Anschluß 36 befindet sich vorteilhafterweise in Nachbarschaft zum ersten Anschluß 18.

Der Aufnahmebereich 14 des männlichen Konnektorstückes ist durch eine Öffnung 40 in Richtung auf das einzuführende weibliche Konnektorstück 4 hin geöffnet. Weiterhin weist die Hülse 21 des männlichen Konnektorstücks 6 zwei Bereiche mit verschiedenen Innendurchmessern auf, wobei ein erster Hülsenbereich 42 einen Innendurchmesser aufweist, der größer ist als der Außendurchmesser des Einführabschnittes 12 der Hülse 10 des weiblichen Konnektorstückes, und ein zweiter Hülsenbereich 44, der sich an den ersten Hülsenbereich 42 anschließt, einen Innendurchmesser aufweist, der wenigstens annähernd gleich, vorzugsweise etwas größer als der Außendurchmesser des Einführabschnittes 12 des weiblichen Konnektorstückes 6 ist.

Im Bereich der Öffnung 40 des männlichen Konnektorstückes 6 springt von dem ersten Hülsenbereich 42 wenigstens einer, in der dargestellten Ausführungsform zwei Bolzen 46 radial nach innen vor. Die radiale Erstreckung der Bolzen 46 nach innen, sowie deren Außendurchmesser ist derart bemessen, daß ein formschlüssiger Eingriff mit einer Führungsnut 48, die in einem Führungsabschnitt der ersten Hülse 10 vorgesehen ist, wie später noch unter Bezugnahme auf Fig. 3 bis 7 näher erläutert wird, möglich ist.

Im Inneren des weiblichen Konnektorstückes 4 ist eine Ventileinrichtung 52 angeordnet. Diese Ventileinrichtung 52 weist im wesentlichen eine ringförmige Dichtung 54 auf, welche aus einem geeigneten elastomeren Material besteht, und in einer ringförmigen Ausnehmung 56 in der Innenwand des Einführabschnitts 12 der ersten Hülse 10 gehalten ist. Die Halterung der Dichtung 54 in der Ausnehmung 56 erfolgt durch Verkleben, Preßsitz, einen Sprengring oder dergleichen.

Der Durchmesser der Öffnung 57 der Dichtung 54 ist hierbei wenigstens annähernd gleich, vorzugsweise etwas geringer als ein Außendurchmesser D des Hauptabschnitts 28 des Rohrstücks 22 im männlichen Konnektorstück 6. Andererseits ist der Durchmesser der Öffnung 57 größer als der Außendurchmesser E des Frontabschnitts 30 des Rohrstücks 22.

weiterhin weist die Ventileinrichtung 52 im Konnektorstück 4 eine Ventilplatte 58 auf, welche mittels einer geeigneten Federeinrichtung, beispielsweise durch eine Spiral-Druckfeder 60 in flüssigkeitsdichter Anlage mit der ringförmigen Dichtscheibe 54 gehalten ist. Die Ventilplatte 58 ist dabei zwischen der Dichtung 54 und dem zweiten Anschluß 32 im weiblichen Konnektorstück 4 angeordnet.

Die Druckfeder 60 stützt sich mit ihrem einen Ende an einem geeigneten Federsitz 62 ab, und eine geeignete Ausbildung der der Feder zugewandten Kontur der Ventilplatte 58 erlaubt eine formschlüssige Verbindung zwischen der Ventilplatte 58 und der Druckfeder 60. Im Bereich zwischen der Ventilplatte 58 und dem Federsitz 62 ist an der Innenkontur des weiblichen Konnektorstückes 4 ein Anschlag 64 in Form eines vorspringenden Bereiches ausgebildet.

Im äußeren Endbereich des Einführabschnitts 12 des weiblichen Konnektorstücks 4, vorzugsweise benachbart zur Einführöffnung 65, ist ein ringförmiges Abdichtelement, beispielsweise ein O-Ring 66 angeordnet, der vorteilhafterweise in eine Ringnut eingelassen ist, die sich auf der Außenoberfläche des weiblichen Konnektorstückes 4 befindet.

Im folgenden wird nun die Funktionsweise des vorliegenden Konnektors, insbesondere unter Bezugnahme auf die Fig. 1 und 3 bis 7 beschrieben:
In dem Zustand gemäß Fig. 1, das heißt im nichtkonnektierten Zustand, sind sowohl das weibliche Konnektorstück 4 als auch das männliche Konnektorstück 6 je mit einer in der Zeichnung nicht dargestellten Kappe verschlossen, wobei die von der Kappe verschlossenen Innenräume ggf. mit einem geeigneten Desinfektionsmittel sterilisiert sind. Um einen ersten Konnektionszustand gem. Fig. 3 herzustellen, werden zunächst die beiden Kappen entfernt und das weibliche Konnektorstück 4 wird zu dem männlichen Konnektorstück 6 derart ausgerichtet, daß der oder die Bolzen 46 an dem männlichen Konnektorstück 6 mit einem Einlaufabschnitt 68 der Führungsnut 48 an dem Führungsabschnitt 50 des weiblichen Konnektorstückes 4 fluchten.

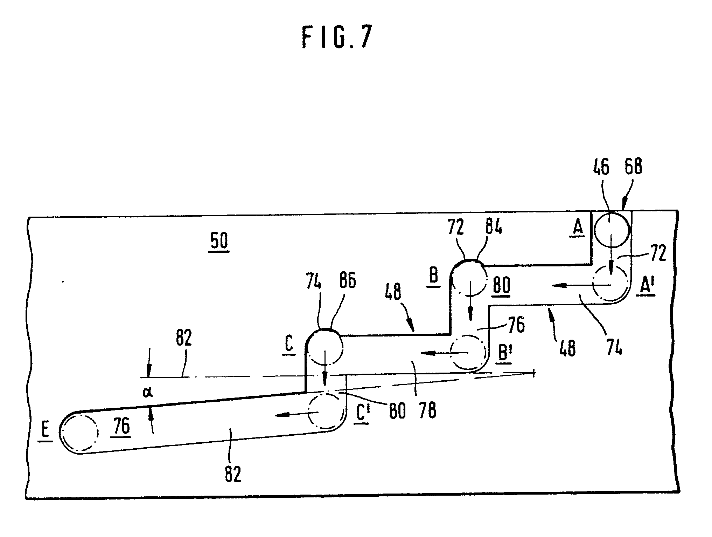

Die Stellung des Bolzens 46 zu der Führungsnut 48 bzw. zu dem Einlauf 68 ist in Fig. 7 mit A gekennzeichnet. Fig. 7 zeigt die Führungsnut 48 in abgewickelter Darstellung und man erkennt, daß es sich um eine 3stufige Bajonettbahn handelt. Wird nun das weibliche Konnektorstück 4 in das männliche Konnektorstück 6 geschoben, erreicht der Bolzen 46 in der Führungsnut 48 zunächst eine erste Kupplungsstellung, die in Fig. 7 mit A' gekennzeichnet ist. In dieser Kupplungsstellung hat das weibliche Konnektorstück 4 in dem männlichen Konnektorstück 6 eine Kupplungsstellung gem. Fig. 3 erreicht, in welcher der O-Ring abdichtend an der inneren Umfangsoberfläche des zweiten Hülsenbereiches 44 des männlichen Konnektorstückes 6 anliegt. Durch eine entsprechende Drehung des weiblichen Konnektorstückes 4 relativ zu dem männlichen Konnektorstück 6 folgt der Bolzen 46 der Führungsnut 48 und erreicht eine erste Verriegelungsstellung, welche in Fig. 7 mit B gekennzeichnet ist und in der die erste Kupplungsstellung gesichert ist. In dieser ersten Kupplungsstellung, die in Fig. 3 gezeigt ist und im folgenden Spülstellung genannt wird, bleibt die Ventilplatte 58 der Ventileinrichtung 52 weiterhin aufgrund der Druckbelastung durch die Feder 60 in flüssigkeitdichter Anlage mit der Dichtscheibe 54, so daß nur eine Fluidpassage von dem ersten Anschluß 18 durch das zentrale Rohrstück 22 zu dem dritten Anschluß 36 möglich ist. In dieser Spülstellung wird nun der volle Beutel mit der frischen Dialysierflüssigkeit - beispielsweise durch Brechen eines Brechkonus - geöffnet, und frische Dialysierflüssigkeit strömt durch den ersten Schlauch 20, das zentrale Rohrstück 22 und den dritten Schlauch 38 in den leeren Beutel. Die einströmende frische Dialysierflüssigkeit entfernt sowohl das Desinfektionsmittel, das sich in diesen Bereichen des männlichen bzw. weiblichen Konnektorstückes befindet, als auch Partikel, die sich beim Öffnen des Brechkonus bilden können, und spült sie in den Leerbeutel.

Nach dem Spülen des Konnektors erfolgt als zweiter Schritt bei der Peritonealdialyse das Ablassen der verbrauchten Dialysierflüssigkeit aus dem Peritonalraum des Patienten in den Leerbeutel.

Hierzu wird das weibliche Konnektorstück 4 weiter in das männliche Konnektorstück 6 eingeführt, wobei der Bolzen 46 in der Führungsnut 48 eine zweite Kupplungsstellung erreicht, die in Fig. 7 mit B' gekennzeichnet ist. Ein Verdrehen des weiblichen Konnektorstückes 4 relativ zu dem männlichen Konnektorstück 6 bringt den Bolzen 46 in eine Lage, die in Fig. 7 mit C gekennzeichnet ist, die der Kupplungsstellung gem. Fig. 4 entspricht und in der die zweite Kupplungsstellung gesichert ist.

Ein Einschieben des weiblichen Konnektorstückes 4 in das männliche Konnektorstück 6 bewirkt zunächst, daß die Mündungsöffnung 24 des Rohrstückes 22 in Anlage mit einer Oberfläche 70 der Ventilplatte 58 gerät, so daß der Zulauf von frischer Dialysierflüssigkeit aus dem vollen Beutel durch den ersten Schlauch 20 unterbrochen wird. Ein Weiterbewegen des weiblichen Konnektorstückes 4 in das männliche Konnektorstück 6 hinein (wobei der Bolzen 46 in die Position B' gemäß Fig. 7 gerät) führt schließlich zu der zweiten Kupplungsstellung gemäß Fig. 4. Ist diese zweite Kupplungsstellung erreicht, wird das weibliche Konnektorstück gedreht, so daß der Bolzen 46 in der Führungsnut 48 die Lage C in Fig. 7 einnimmt, in welcher diese zweite Kupplungsstellung verriegelt ist.

Wie aus Fig. 4 hervorgeht, wird in dieser zweiten Kupplungsstellung die Ventilplatte 58 gegen die Federkraft der Druckfeder 60 von dem zentralen Rohrstück 22 aus der Anlage mit der Dichtscheibe 54 bewegt, so daß eine Fluidpassage von dem zweiten Anschluß 32 des weiblichen Konnektorstükkes an der Ventilplatte 58 vorbei und durch den ringförmigen Freiraum 71, der zwischen der Öffnung 57 der ringförmigen Dichtscheibe 54 und der Außenoberfläche des Frontabschnittes 30 des zentralen Rohrstücks 22 erzeugt wird zu dem dritten Anschluß 36 frei wird. Durch diese Fluidpassage strömt die verbrauchte Dialysierflüssigkeit aus dem Peritonealraum des Patienten in den Leerbeutel, wie in Fig. 4 durch die Strömungspfeile angedeutet.

Die Oberfläche 70 der Ventilplatte 58 wird hierbei durch die Druckfeder 60 gegen die Mündungsöffnung 24 des Rohrstücks 22 gepreßt, so daß keine frische Dialysierflüssigkeit aus dem vollen Beutel einströmen kann. Diese zweite Kupplungsstellung wird auch als Ablaufstellung bezeichnet.

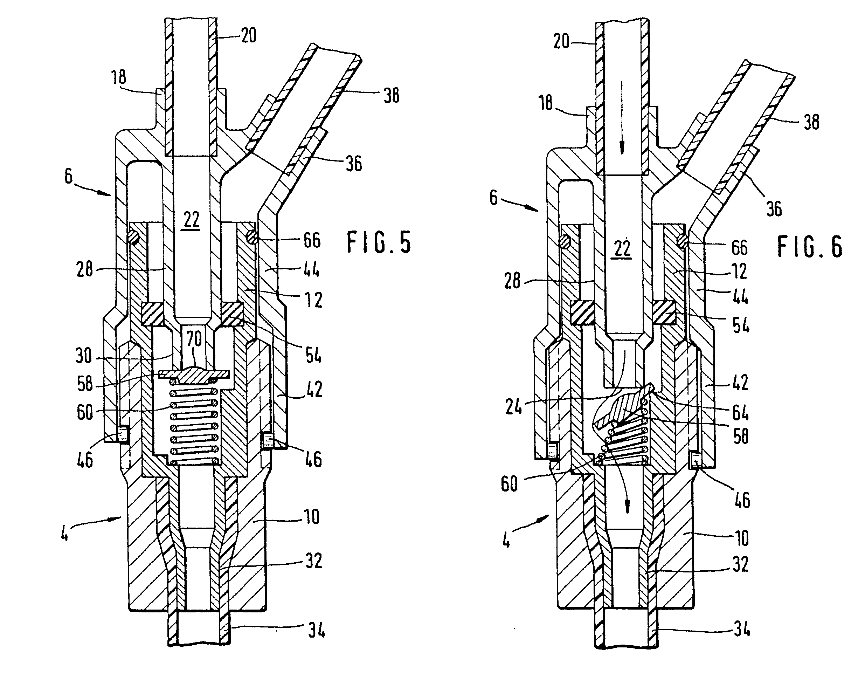

Nachdem die verbrauchte Dialysierflüssigkeit aus dem Peritonealraum des Patienten vollständig in den Leerbeutel abgeflossen ist, erfolgt der dritte Schritt des Zuführens von frischer Dialysierflüssigkeit aus dem vollen Beutel in den Peritonealraum. Hierzu wird zunächst das weibliche Konnektorstück aus dem zweiten Konnektionszustand gem. Fig. 4 in eine dritte Kupplungsstellung gemäß Fig. 5 gebracht, wobei sich der Bolzen 46 gemäß Fig. 7 aus der Position C in die Pos C' bewegt. In dieser dritten Kupplungsstellung, die auch als Zwischenstellung bezeichnet wird, bleibt die Mündungsöffnung 24 des Innenrohrs 22 weiterhin von der Oberfläche 70 der Ventilplatte 58 verschlossen, so daß kein frisches Dialysat aus dem ersten Schlauch 20 zufließen kann. Weiterhin verschließt der Hauptabschnitt 28 des Rohrstücks 22 in diesem dritten Konnektionszustand die Öffnung 57 der ringförmigen Dichtscheibe 54 und somit den ringförmigen Freiraum 71, so daß keinerlei Fluidpassagen zwischen dem ersten, zweiten und dritten Anschluß bestehen. Von wesentlicher Bedeutung hierbei ist, daß das Material, aus dem die Dichtung 54 gefertigt ist, ein Elastomer ist, so daß eine zuverlässige flüssigkeitsdichte Anlage zwischen dem Hauptabschnitt 28 des Rohrstücks 22 und der ringförmigen Dichtscheibe 54 besteht.

Um den Einlaß von frischer Dialysierflüssigkeit aus dem vollen Beutel in den Peritonealraum zu ermöglichen, wird das weibliche Konnektorstück 4 relativ zu dem männlichen Konnektorstück 6 gedreht, so daß der Bolzen 46 aus der Position C' in Fig. 7 in eine Position E in Fig. 7 gelangt. Wie aus Fig. 7 hervorgeht, ist der Bahnverlauf der Führungsnut 48 von der Position C' bis zur Position E nicht mehr parallel zu den beiden Bahnverläufen von A' bis B bzw. von B' bis C, sondern ist hierzu unter einem Winkel α abwärts geneigt, wobei α vorteilhafterweise etwa 5 Grad beträgt. Wenn somit das weibliche Konnektorstück 4 aus der dritten Kupplungsstellung gemäß Fig. 5 gedreht wird, erfährt der Bolzen 46 in der Führungsnut 48 eine Gewindeführung, was zur Folge hat, daß das weibliche Konnektorstück 4 linear und gleichmäßig in das männliche Konnektorstück 6 eingeschraubt wird, bis der Bolzen 46 die Position E in Fig. 7 erreicht hat. Hat der Bolzen 46 die Position E in Fig. 7 erreicht, ist eine vierte Kupplungsstellung oder Einlaufstellung gemäß Fig. 6 erreicht.

Bei der gleichmäßigen Vorschubbewegung des weiblichen Konnektorstückes 4 in das männliche Konnektorstück 6 hinein wird die Ventilplatte 58 zunächst weiter gegen die Kraft der Druckfeder 60 bewegt, wobei einerseits die Mündungsöffnung 24 des Rohrstücks 22 von der Oberfläche 70 der Ventilplatte 58 verschlossen bleibt und andererseits die Außenoberfläche des Hauptabschnitts 28 des Rohrstücks 22 flüssigkeitsdicht mit der Öffnung 57 der Dichtscheibe 54 in Anlage bleibt. Im Zuge der weiteren Bewegung des weiblichen Konnektorstückes 4 in das männliche Konnektorstück 6 hinein gerät die Ventilplatte 58 einseitig in Anlage mit dem Anschlag 64 in dem weiblichen Konnektorstück 4, so daß sie bei einer weiteren Bewegung des weiblichen Konnektorstückes 4 in das männliche Konnektorstück 6 hinein aus ihrer horizontalen Lage gemäß Fig. 1, 3, 4 und 5 unter gleichzeitiger Deformierung der Druckfeder 60 gekippt wird, wie in Fig. 6 dargestellt. Durch dieses Kippen der Ventilplatte 58 wird die Mündungsöffnung 24 des Rohrstücks 22 kontinuierlich freigegeben, so daß ein Fluiddurchlaß von dem ersten Anschluß 18 durch das Rohrstück 22, die Mündungsöffnung 24 und durch die Wicklung der Feder 60 hindurch zu dem zweiten Anschluß 32 freigegeben wird. Durch diesen Fluiddurchlaß kann nun frische Dialysierflüssigkeit aus dem vollen Beutel in den peritonealraum des Patienten einströmen, wie in Fig.6 durch die Strömungspfeile dargestellt.

Bei einer entsprechenden Ausgestaltung sowohl der Oberfläche 70 der Ventilplatte 58 als auch des Neigungswinkels des letzten Ganges der Führungsnut 48 kann ein linear gleichmäßiges Öffnen der Mündungsöffnung 24 erzielt werden, so daß die Dialysierflüssigkeit aus dem vollen Beutel mit kontinuierlich wachsender Strömungsgeschwindigkeit und mit kontinuierlich wachsendem Strömungsvolumen in den Peritonealraum einströmt.

Nachdem die Dialysierflüssigkeit aus dem vollen Beutel in den Peritonealraum des Patienten abgeflossen ist, werden die bisher beschriebenen Schritte rückwärts ausgeführt, das heißt, zunächst wird das weibliche Konnektorstück 4 relativ zu dem männlichen Konnektorstück 6 gedreht, um in die dritte Kupplungsstellung gem. Fig. 5 zu gelangen, in welchem der Zugang zu dem Peritonealraum durch die Ventilplatte 58, welche die Mündungsöffnung 24 des Rohrstückes 22 verschließt, wieder verschlossen ist. Durch entsprechende Dreh- und Zugbewegungen erfolgt sodann die Diskonnektion des weiblichen Konnektorstückes 4 von dem männlichen Konnektorstück 6, wobei nacheinander die Kupplungsstellungen gemäß den Fig. 4 und 3 durchlaufen werden, bis wieder der Diskonnektionszustand gemäß Fig.1 erreicht ist.

Das offene Ende des weiblichen Konnektorstückes 4 wird nun wieder mit einem geeigneten Desinfektionsmittel eingesprüht und mit einer Kappe verschlossen und das männliche Konnektorstück 6, das mit dem vollen Beutel und dem Leerbeutel,in welchem sich nun die verbrauchte Dialysierflüssigkeit befindet, wird als Einheit vernichtet.

Abschließend soll anhand von Fig. 7 der genaue Aufbau der Führungsnut 48 in dem Führungsabschnitt 50 erläutert werden:

Wie bereits erwähnt, stellt die Führungsnut 48 eine dreistufige Bajonettbahn dar, so daß der Bolzen 46 während seines Verlaufes in der Führungsnut 48 bewirkt, daß das weibliche Konnektorstück 4 relativ zu dem männlichen Konnektorstück 6 Hub- und Drehbewegungen ausführt. Infolge dessen besteht die Führungsnut 48 aus folgenden Abschnitten:

An einen ersten Hubabschnitt 72 folgt ein erster Drehabschnitt 74, von dem sich aus ein zweiter Hubabschnitt 76 erstreckt, der wiederum in einen zweiten Drehabschnitt 78 übergeht, der sich in einen dritten Hubabschnitt 80 fortsetzt. Von diesem dritten Hubabschnitt 80 erstreckt sich ein Schraubabschnitt 82, der eine kombinierte Dreh-/Hubbewegung zuläßt.

Während die Hubabschnitte 72, 76 und 80 im wesentlichen parallel zur Konnektionsachse ausgerichtet sind, stehen die Drehabschnitte 74 und 78 im wesentlichen senkrecht auf der Konnektionsachse. Dagegen ist der Schraubabschnitt 82 gegenüber der Konnektionsachse um den vorstehend erwähnten Winkel α geneigt.

Um die Lage des Bolzens 46 am Punkt B zu sichern, ist der erste Drehabschnitt 74 an seinem Schnittpunkt mit dem zweiten Hubabschnitt 76 vorteilhafterweise mit einer Rast 84 versehen, in welcher der Bolzen 46 eine gesicherte Ruhelage einnehmen kann, so daß die erste Kupplungsstellung gem. Fig. 3 gesichert ist.

In ähnlicher Weise ist die zweite Kupplungsstellung gem. der Lage C von Fig. 4 dadurch zu sichern, daß der zweite Drehabschnitt 78 an seinem Schnittpunkt mit dem dritten Hubabschnitt 80 ebenfalls eine ausgeformte Rast 86 aufweist, die den Bolzen 46 in der zweiten Kupplungsstellung sichert. Da in dieser Stellung gem. Fig. 4 die Druckfeder 60 bereits um einen gewissen Betrag komprimiert ist, wird der Bolzen 46 von der Federkraft, welche das weibliche Konnektorstück 4 vom männlichen Konnektorstück 6 zu trennen versucht, in diese zweite Rast 76 gepreßt und dort sicher gehalten.

Wie bereits erwähnt, ist der Schraubabschnitt 82 der Bajonettbahn gegenüber der Konnektionsachse des Konnektors 2 um einen Winkel α geneigt. Aufgrund der Federkraft Druckfeder 60 wird der Bolzen 46 in seiner endgültigen Endlage bei E gegen die Bajonettbahn gedrückt und dort ohne eine weitere Rast nur durch Reibschluß gehalten.

Zusammenfassend ist somit festzuhalten, daß mit dem bisher beschriebenen Konnektor eine rasche und gründliche Spülung des Konnektors vor dem Dialysiervorgang mit einem Minimum an Dialysierflüssigkeit möglich ist, und weiterhin auf den Einsatz von Rollenklemmen in den entsprechenden Zufuhrleitungen vollständig verzichtet werden kann, da das Öffnen und Schließen der entsprechenden Fluidpassagen einzig durch Bewegung des Konnektors selbst erfolgt. Eine erhöhte Betriebssicherheit, insbesondere bei der CAPD, ist somit in jedem Fall gewährleistet.

Die Auswahl der Materialien zum Herstellen des erfindungsgemäßen Konnektors liegt im Bereich fachmännischen Handelns - es kommen physiologisch unbedenkliche Metalle und/oder Kunststoff in Frage.

## Patentansprüche

1. Konnektor (2) für eine CAPD-Schlauchverbindung mit einem männlichen Konnektorstück (6), das ein zentrales Rohrstück (22) männlichen Konnektorstück (6), das ein zentrales Rohrstück (22) aufweist, das von einer Schutzhülse (21) umfaßt und übergriffen ist, und einem weiblichen Konnektorstück (4), das eine Ventileinrichtung (52) im Einführungsabschnitt (12) aufweist, welche im ungekuppelten Zustand eine Fluidsperre darstellt, wobei der Einführungsabschnitt (12) in einen Aufnahmebereich (14) der Schutzhülse (21) mit einer Dichtung (66) zwischen der Schutzhülse (21) und dem weiblichen Konnektorstück (4) flüssigkeitsdicht einführbar ist, mit einem ersten Anschluß (18) am männlichen Konnektorstück (6) für einen vollen Beutel mit Dialysierflüssigkeit, wobei der erste Anschluß (18) mit dem zentralen Rohrstück (22) in Fluidverbindung steht, mit einem zweiten Anschluß (32) am weiblichen Konnektorstück (4), an welchem ein Peritonealkatheter anschließbar ist, und mit einer Verbindungseinrichtung (46,48) zur Sicherung einer Konnektionsstellung zwischen dem weiblichen (4) und dem männlichen Konnektorstück (6), dadurch gekennzeichnet, daß am männlichen Konnektorstück (6) im Bereich des ersten Anschlusses (18) an der Schutzhülse (21) ein dritter Anschluß, (36) für einen Leerbeutel für verbrauchte Dialysierflüssigkeit angeordnet ist und daß die Ventileinrichtung (52) in Abhängigkeit von der Konnektionsstellung des weiblichen Konnektorstücks (4) relativ zu dem männlichen Konnektorstück (6) und im Zusammenwirken mit dem zentralen Rohrstück (22) des männlichen Konnektorstücks (6) Fluidpassagen zwischen dem ersten, zweiten und dritten Anschluß (18, 32, 36) derart freigibt, daß entweder der erste Anschluß (18) mit dem zweiten Anschluß (32) oder der erste Anschluß (18) mit dem dritten Anschluß (36) oder der zweite Anschluß (32) mit dem dritten Anschluß (36) verbanden wird, während gleichzeitig jeweils die Fluidverbindung zum übrigen Anschluß unterbrochen wird.

2. Konnektor nach Anspruch 1, **dadurch gekennzeichnet**, daß die jeweilige Konnektionsstellung durch die Verbindungseinrichtung festgelegt ist.

3. Konnektor nach Anspruch 2, **dadurch gekennzeichnet**, daß die Verbindungseinrichtung wenigstens einen Bolzen (46) am männlichen Konnektorstück (6) aufweist, welcher in eine entsprechend ausgebildeten Führungsnut (48) am weiblichen Konnektorstück (4) eingreift.

4. Konnektor nach Anspruch 3, **dadurch gekennzeichnet**, daß die Führungsnut (48) stufenförmig entlang der Umfangrichtung Hülse (10) des weiblichen Konnektorstückes (4) verläuft.

5. Konnektor nach Anspruch 4, **dadurch gekennzeichnet**, daß die Führungsnut (48) drei Hubabschnitte (72, 76, 80) zwei mit dem ersten und zweiten bzw. zweiten und dritten Hubabschnitt (72, 76, 80) verbundenen Drehabschnitte (74, 78) und einem sich von dem dritten Hubabschnitt (80) erstreckenden Schraubabschnitt (82) aufweist.

6. Konnektor nach Anspruch 5, **dadurch gekennzeichnet**, daß der Schraubabschnitt (82) zur Mittelachse des Konnektors (2) geneigt ist.

7. Konnektor nach Anspruch 1, **dadurch gekennzeichnet**, daß die Ventileinrichtung (52) eine Ventilplatte (58) aufweist, welche unter Federbelastung an einer ringförmigen Dichtung (54) fluiddicht anliegt.

8. Konnektor nach Anspruch 7, dadurch gekennzeichnet, daß die Ventilplatte (58) und die ringförmige Dichtung (54) aus elastomerem Material gefertigt sind.

9. Konnektor nach Anspruch 7, dadurch gekennzeichnet, daß die Ventilplatte (58) durch eine Druckfeder (60) in Richtung auf die ringförmige Dichtung (54) vorgespannt ist.

10. Konnektor nach Anspruch 7, dadurch gekennzeichnet, daß die Ventilplatte (58) mit dem zentralen Rohrstück (22) aus ihrer Anlage mit der ringförmigen Dichtung (54) aushebbar ist.

11. Konnektor nach Anspruch 10, dadurch gekennzeichnet, daß die Aushubbewegung der Ventilplatte (58) durch einen auf einen Randbereich der Ventilplatte (58) wirkenden Anschlag (64) innerhalb des weiblichen Konnektorstückes (4) in eine Kippbewegung überführbar ist.

12. Konnektor nach Anspruch 1, dadurch gekennzeichnet, daß das weibliche Konnektorstück (4) benachbart zu seinem Einführabschnitt (12) ein ringförmiges Abdichtteil aufweist.

13. Konnektor nach Anspruch 12, dadurch gekennzeichnet, daß das Abdichtteil ein O-Ring (66) ist.

14. Konnektor nach Anspruch 1, dadurch gekennzeichnet, daß das zentrale Rohrstück (22) einen Frontabschnitt (30) mit einem Außendurchmesser E und einem Hauptabschnitt (28) mit einem Außendurchmesser D aufweist, der größer ist als der Außendurchmesser E und daß der Außendurchmesser D des Hauptabschnitts (28) wenigstens annähernd gleich, vorzugsweise geringfügig größer als der Durchmesser d der ringförmigen Öffnung (57), und daß der Außendurchmesser E des Frontabschnitts (30) kleiner ist als der Durchmesser d der Öffnung (57).

15. Konnektor nach Anspruch 1, dadurch gekennzeichnet, daß das männliche Konnektorstück (6) in einer ersten Kupplungsstellung mit dem weiblichen Konnektorstück (4) fluiddicht mit der ringförmigen Dichtung (66) zusammenwirkt und eine erste Fluidpassage vom ersten Anschluß (18) zum dritten Anschluß (36) bewirkt, daß in einer zweiten Kupplungsstellung durch das zentrale Rohrstück (22) des männlichen Konnektorstücks (6) die Ventileinrichtung (52) des weiblichen Konnektorstücks (4) geöffnet ist, wobei die Mündungsöffnung (26) des Rohrstücks (22) fluiddicht an der Ventilplatte (58) anliegt und der Frontabschnitt (30) in der Öffnung (57) der ringförmigen Dichtung (56) einen ringförmigen Freiraum erzeugt, der eine zweite Fluidpassage vom zweiten Anschluß (32) zum dritten Anschluß (36) bewirkt, daß in einer dritten Kupplungsstellung der Hauptabschnitt (28) des zentralen Rohrstücks (22) des männlichen Konnektorstücks (6) fluiddicht mit der ringförmigen Dichtung (56) des weiblichen Konnektorstücks (4) zusammenwirkt und die Ventilplatte (58) von der Mündungsöffnung (24) des Rohrstücks (22) unter Bildung einer dritten Fluidpassage vom ersten Anschluß (18) zum zweiten Anschluß (32 ) gekippt ist und daß die Kupplungsstellungen durch die Verbindungseinrichtung vorgegeben und verrastbar sind.

**Claims**

1. Connector (2) for a CAPD tube connection device, comprising a male connector (6) piece having a central tube section (22) which is engaged and surrounded by a protective sleeve (21), and a female connector piece (4) which comprises a valve means (52) in the introducing portion (12) which in the uncoupled state represents a fluid barrier, the introduction portion (12) being introducible in liquid-tight manner into a receiving region (14) of the protection sleeve (21) with a seal (66) between the protective sleeve (21) and the female connector piece (4), a first connection (18) on the male connector piece (6) for a full bag with dialysis solution, the first connection (18) being in fluid connection with the central tube section (22), a second connection (32) on the female connector piece (4) to which a peritoneal catheter is connectable, and a connecting means (46, 48) for securing a connection position between the female (4) and the male connector piece (6), characterized in that on the male connector piece (6) in the region of the first connection (18) on the protective sleeve (21) a third connection (36) is disposed for an empty bag for used dialysis solution and that the valve means (52) in dependence upon the connection position of the female connector piece (4) relatively to the male connector piece (6) and in cooperation with the central tube section (22) of the male connector piece (6) free fluid passages between the first, second and third connections (18, 32, 36) such that either the first connection (18) is connected with the second connection (32) or the first connection (18) with the third connection (36) or the second connection (32) with the third connection (36) whereas simultaneously the fluid connection to the other connection is interrupted.

2. Connector according to claim 1, characterized in that the respective connection position is secured by the connecting means.

3. Connector according to claim 2, characterized in that the connecting means comprises at least one pin (46) on the male connector piece (6) which engages in a correspondingly formed guide groove (48) on the female connector piece (4).

4. Connector according to claim 3, characterized in that the guide groove (48) extends in stepped manner along the peripheral direction of the sleeve (10) of the female connector piece (4).

5. Connector according to claim 4, characterized in that the guide groove (48) comprises three lifting portions (72, 76, 80), two rotary portions (74, 78) connected to the first and second and second and third lifting portions (72, 76, 80) respectively and a screw portion (82) extending from the third lifting portion (80).

6. Connector according to claim 5, characterized in that the screw portion (82) is inclined to the centre axis of the connector (2).

7. Connector according to claim 1, characterized in that the valve means (52) comprises a valve plate (58) which under spring loading bears on an annular seal (54) in fluid-tight manner.

8. Connector according to claim 7, characterized in that the valve plate (58) and the annular seal (54) are made from elastomeric material.

9. Connector according to claim 7, characterized in that the valve plate (58) is biased by a pressure spring (60) in the direction towards the annular seal (54).

10. Connector according to claim 7, characterized in that the valve plate (58) is adapted with the central tube section (22) to be lifted out of its engagement with the annular seal (54).

11. Connector according to claim 10, characterized in that the lifting movement of the valve plate (58) can be converted to a tilting movement by a stop (64) acting on an edge region of the valve plate (58) within the female connector piece (4).

12. Connector according to claim 1, characterized in that the female connector piece (4) comprises an annular sealing member adjacent its introduction portion (12).

13. Connector according to claim 12, characterized in that the sealing member is an O ring (66).

14. Connector according to claim 1, characterized in that the central tube section (22) comprises a front portion (30) with an external diameter E and a main portion (28) with an external diameter D which is greater than the external diameter E and that the external diameter D of the main portion (28) is at least approximately equal to, preferably slightly greater than, the diameter d of the annular opening (57) and that the outer diameter E of the front portion (30) is less than the diameter d of the opening (57).

15. Connector according to claim 1, characterized in that the male connector piece (6) cooperates in a first coupling position with the female connector piece (4) in fluid-tight manner with the annular seal (66) and effects a first fluid passage from the first connector (18) to the third connection (36), that in a second coupling position by the central tube section (22) of the male connector piece (6) the valve means (52) of the female connector piece (4) is opened, the mouth opening (26) of the tube section (22) bearing in fluid-tight manner on the valve plate (58) and the front portion (30) forming in the opening (57) of the annular seal (56) an annular free space which effects a second fluid passage from the second connection (32) to the third connection (36),

that in a third coupling position the main portion (28) of the central tube section (22) of the male connector piece (6) cooperates in fluid-tight manner with the annular seal (56) of the female connector piece (4) and the valve plate (58) is tilted from the mouth opening (24) of the tube section (22) to form a third fluid passage from the first connection (18) to the second connection (32) and that the coupling positions are predetermined and lockable by the connecting means.

## Revendications

1. Raccord (2) pour un ensemble de tuyaux flexibles pour DPCA (dialyse péritonéale ambulatoire continue) comportant une pièce de raccord mâle (6) qui présente un bout de tube central (22) entouré et recouvert d'un manchon de protection (21) et une pièce de raccord femelle (4) présentant un dispositif à soupape (52) dans sa section d'introduction (12) qui, dans l'état de non-raccordement, constitue un obturateur hydraulique, la section d'introduction (12) pouvant être introduite de manière étanche aux liquides dans une zone de réception (14) du manchon de protection (21) avec un joint d'étanchéité (66) entre le manchon de protection (21) et la pièce de raccord femelle (4), comportant une première tubulure (18) sur la pièce de raccord mâle (6) destinée a une poche remplie de liquide de dialyse, la première tubulure (18) étant en communication d'écoulement avec le bout de tube central (22), une deuxième tubulure (32) sur la pièce de raccord femelle (4) à laquelle peut être raccordé un cathéter péritonéal et un dispositif d'assemblage (46, 48) servant à verrouiller une position de raccordement entre la pièce de raccord femelle et mâle, caractérisé en ce que, sur la pièce de raccord mâle (6), dans la zone de la première tubulure (18), sur le manchon de protection (21), est prévue une troisième tubulure (36) destinée à une poche vide pour le liquide de dialyse usé et que le dispositif à soupape (52), en fonction de la position de raccordement de la pièce de raccord femelle (4) par rapport à la pièce de raccord mâle (6) et en coopération avec le bout de tube central (22) de la pièce de raccord mâle (6), libère des passages d'écoulement entre la première, la deuxième et la troisième tubulure (18, 32, 36), d'une manière telle que la première tubulure (18) est raccordée à la deuxième tubulure (32) ou la première (18) à la troisième (36) ou encore la deuxième (32) à la troisième (36), tandis que, simultanément, le raccordement à la tubulure restante est chaque fois interrompu.

2. Raccord suivant la revendication 1, caractérisé en ce que chaque position de raccordement est déterminée par le dispositif d'assemblage.

3. Raccord suivant la revendication 2, caractérisé en ce que le dispositif d'assemblage comporte au moins un ergot (46) prévu sur la pièce de raccord mâle (6), qui s'engage dans une rainure de guidage (48) de configuration adéquate prévue sur la pièce de raccord femelle (4).

4. Raccord suivant la revendication 3, caractérisé en ce que la rainure de guidage (48) s'étend en gradins le long de la périphérie de la douille (10) de la pièce de raccord femelle (4).

5. Raccord suivant la revendication 4, caractérisé en ce que la rainure de guidage (48) comporte trois sections de levée (72, 76, 80), deux sections de rotation (74, 78) raccordées respectivement aux première et deuxième et aux deuxième et troisième sections de levée (72, 76, 80) et une section de vissage (82) partant de la troisième section de levée (80).

6. Raccord suivant la revendication 5, caractérisé en ce que la section de vissage (82) est inclinée par rapport à l'axe central du raccord (2).

7. Raccord suivant la revendication 1, caractérisé en ce que le dispositif à soupape (52) comprend une soupape (58), qui, sous l'effet d'une sollicitation par ressort, est appliquée de manière étanche contre un joint d'étanchéité annulaire (54).

8. Raccord suivant la revendication 7, caractérisé en ce que la soupape (58) et le joint d'étanchéité annulaire (54) sont faits d'une matière élastomère.

9. Raccord suivant la revendication 7, caractérisé en ce que la soupape (58) est rappelée par un ressort de pression (60) en direction du joint d'étanchéité annulaire (54).

10. Raccord suivant la revendication 7, caractérisé en ce que la soupape (58) peut être soulevée depuis sa position en contact avec le joint d'étanchéité annulaire (54) par le bout de tube central (22).

11. Raccord suivant la revendication 10, caractérisé en ce que le mouvement de levée de la soupape (58) peut être transformé en un mouvement de basculement par une butée (64) agissant sur une zone marginale de la soupape (58) à l'intérieur de la pièce de raccord femelle (4).

12. Raccord suivant la revendication 1, caractérisé en ce que la pièce de raccord femelle (4) présente, à proximité de sa section d'introduction (12), un élément d'étanchéité annulaire.

13. Raccord suivant la revendication 12, caractérisé en ce que l'élément d'étanchéité est un anneau torique (66).

14. Raccord suivant la revendication 1, caractérisé en ce que le bout de tube central (22) comporte une section antérieure (30) ayant un diamètre extérieur E et une section principale (28) ayant un diamètre extérieur D qui est supérieur au diamètre extérieur E et que le diamètre extérieur D de la section principale (28) est au moins approximativement égal, de préférence légèrement supérieur au diamètre d de l'orifice de joint annulaire (57) et que le diamètre extérieur E de la section antérieure (30) est inférieur au diamètre d de l'orifice (57).

15. Raccord suivant la revendication 1, caractérisé en ce que la pièce de raccord mâle (6), dans une première position de raccordement avec la pièce de raccord femelle (4), coopère de manière étanche avec le joint d'étanchéité annulaire (66) et établit un premier passage d'écoulement allant de la première tubulure (18) à la troisième (36), que, dans une deuxième position de raccordement, le dispositif à soupape (52) de la pièce de raccord femelle (4) est ouvert par le bout de tube central (22) de la pièce de raccord mâle (6), l'embouchure (26) du bout de tube (22) étant appliquée de manière étanche contre la soupape (58) et la section antérieure (30) dans l'orifice (57) du joint d'étanchéité annulaire (56) créant un espace annulaire qui établit un deuxième passage d'écoulement allant de la deuxième tubulure (32) à la troisième tubulure (36), que, dans la troisième position de raccordement, la section principale (28) du bout de tube central (22) de la pièce de raccord mâle (6) coopère de manière étanche avec le joint d'étanchéité annulaire (56) de la pièce de raccord femelle (4) et la soupape (58) est écartée par basculement de l'embouchure (24) du bout de tube central (22) avec formation d'un troisième passage d'écoulement allant de la première tubulure (18) à la deuxième tubulure (32) et que les positions de raccordement sont prédéfinies et peuvent être verrouillées par le dispositif d'assemblage.

11

EP 0 198 407 B1

FIG.1

FIG.2

EP 0 198 407 B1

FIG. 4

FIG. 3

13

FIG. 6

FIG. 5

# FIG.7